(19) Europäisches Patentamt
European Patent Office
Office européen des brevets

(11) Veröffentlichungsnummer: **0 015 882**
**B1**

(12) **EUROPÄISCHE PATENTSCHRIFT**

(45) Veröffentlichungstag der Patentschrift: **16.06.82**

(51) Int. Cl.³: **C 07 C 101/04,** C 07 C 101/28, C 07 C 101/18 // C07D225/02

(21) Anmeldenummer: **80810083.8**

(22) Anmeldetag: **06.03.80**

(54) **Undecansäuren und Ester derselben sowie Verfahren zu ihrer Herstellung.**

(30) Priorität: **12.03.79 CH 2330/79**

(43) Veröffentlichungstag der Anmeldung: **17.09.80 Patentblatt 80/19**

(45) Bekanntmachung des Hinweises auf die Patenterteilung: **16.06.82 Patentblatt 82/24**

(84) Benannte Vertragsstaaten: **CH DE FR GB IT**

(56) Entgegenhaltungen:
**DE-A-2 831 299**
**FR-A-928 265**
**US-A-3 839 370**
**JOURNAL OF THE AMERICAN OIL CHEMISTS' SOCIETY, Band 51, Oktober 1974, Seiten 427–432 Chicago, USA, W.E. MILLER et al.: «9(10)-carboxy-octadecylamine and 9(10)-amino-methyloctadecanoic acid: synthesis and polymerization to polyamides with lateral substitution»**
**EUGEN MULLER: «Methoden der organischen Chemie (Houben-Weyl), vierte Auflage, Band VIII: Sauerstoffverbindungen III, Seiten 407–413**

(73) Patentinhaber: **CIBA-GEIGY AG, Patentabteilung Postfach, CH-4002 Basel (CH)**

(72) Erfinder: **Reinehr, Dieter Dr., Wolfsheule 10, D-7842 Kandern (DE)**
Erfinder: **Lienhard, Paul Dr., Kirschgartenstrasse 14, CH-4402 Frenkendorf (CH)**

**EP 0 015 882 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Undecansäuren und Ester derselben sowie Verfahren zu ihrer Herstellung

Die vorliegende Erfindung betrifft in der Kohlenstoffkette mindestens 2-fach substituierte 11-Amino-undecansäuren und 11-Amino-undeca-4,8-diensäuren, die Ester dieser Säuren sowie die Herstellungsverfahren für diese Verbindungen. Die Verbindungen sind neu und die Ester sind als Ausgangssubstanzen für die Herstellung von hochwertigen Azofarbstoffsalzen geeignet.

In der Kohlenstoffkette mindestens 2-fach substituierte 11-Amino-undecanole und die Herstellung derselben sind bereits in der DE-OS-2 831 299 beschrieben. Auch die unsubstituierte 11-Amino-undecansäure ist bereits in dem «Lehrbuch der organischen Chemie» (Seite 345) von H. Beyer, S. Hirzel Verlag Leipzig 1968, beschrieben.

Bekannte Farbstoffsalze, wie beispielsweise die in der GB-PS-1 296 857 beschriebenen Azofarbstoffalkalisalze, weisen den Nachteil auf, dass ihre Löslichkeit in organischen Lösungsmitteln verhältnismässig stark begrenzt ist.

Die Aufgabe der Erfindung besteht in der Bereitstellung von aliphatischen gesättigten und ungesättigten Aminocarbonsäureestern, welche für die Herstellung von Salzen anionischer Farbstoffe, insbesondere von Azofarbstoffsulfonsäuresalzen, die in ihrer Löslichkeit in organischen Lösungsmitteln den bekannten Salzen anionischer Farbstoffe überlegen sind, geeignet sind. Die Aufgabe umschliesst auch die Bereitstellung der freien Säuren dieser Ester, welche Ausgangsprodukte für letztere sind.

Gegenstand der Erfindung sind Verbindungen der Formel I

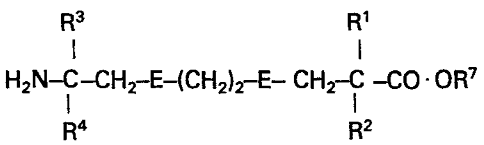 (I)

worin $R^1$ und $R^3$ unabhängig voneinander Wasserstoff oder einen geradkettigen oder verzweigten Alkylrest mit 1–8 C-Atomen, $R_3$ ausserdem einen Phenylrest, $R^2$ einen geradkettigen oder verzweigten Alkylrest mit 1–8 C-Atomen und $R^4$ einen geradkettigen oder verzweigten Alkylrest mit 1–18 C-Atomen darstellen oder $R^1$ und $R^2$ und/oder $R^3$ und $R^4$ zusammen mit dem Bindungs-C-Atom einen cycloaliphatischen Ring mit 4–8 C-Atomen bilden, und worin E jeweils einen der Reste

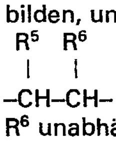 und 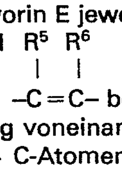 bedeutet, in denen $R^5$ und $R^6$ unabhängig voneinander für Wasserstoff oder Alkyl mit 1–4 C-Atomen stehen und $R^7$ Wasserstoff oder einen geradkettigen oder verzweigten Alkylrest mit insgesamt 1 bis 18 C-Atomen darstellt.

Bevorzugt sind 11-Amino-undecansäuren der Formel I, wobei $R^7$ entsprechend –H und E den Rest 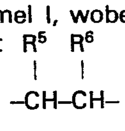 bedeuten.

In ihrer Bedeutung besonders herauszustellen sind auch 11-Amino-undecansäureester der Formel I, in der $R^7$ einen geradkettigen oder verzweigten Alkylrest mit insgesamt 1 bis 18, vorzugsweise 1 bis 8, C-Atomen und E den Rest 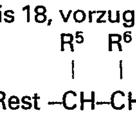 bedeuten, und 11-Amino-undeca-4,8-diensäureester der Formel I, in der $R^7$ ebenfalls einen geradkettigen oder verzweigten Alkylrest mit insgesamt 1 bis 18, vorzugsweise 1 bis 8, C-Atomen und E den Rest 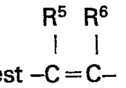 bedeuten.

Eine weitere Vorzugsform der Erfindung stellen Verbindungen der Formel I dar, worin $R^5$ und $R^6$ je Wasserstoff, $R^1$ und $R^3$ unabhängig voneinander Wasserstoff oder Alkyl mit 1–5 C-Atomen, $R^2$ Alkyl mit 1–5 C-Atomen und $R^4$ Alkyl mit 1–7 C-Atomen darstellen, oder worin $R^3$, $R^5$ und $R^6$ je Wasserstoff, $R^1$ und $R^2$ zusammen mit dem Bindungs-C-Atom Cyclopentyl oder Cyclohexyl und $R^4$ Alkyl mit 1–7 C-Atomen bedeuten.

Weitere bevorzugte Verbindungen stellen solche der Formel I dar, worin $R^3$, $R^5$ und $R^6$ je Wasserstoff, $R^1$ und $R^2$ unabhängig voneinander Alkyl mit 1–4 C-Atomen oder zusammen mit dem Bindungs-C-Atom Cyclohexyl und $R^4$ Alkyl mit 1–7 C-Atomen darstellen. Bevorzugt sind auch solche Verbindungen der Formel I, in der $R^3$ H und $R^4$ den Rest 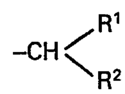 bedeuten.

Besonders bevorzugt ist eine Verbindung der Formel I, worin $R^1$ und $R^2$ je $-CH_3$, $R^3$ –H, $R^4$ $-CH(CH_3)_2$, E $-CH_2CH_2-$ und $R^7$ n-Pentyl bedeuten.

Die Herstellung der erfindungsgemässen Verbindungen der Formel I erfolgt in der Weise, dass man ein 1-Aza-cyclododecan der Formel II

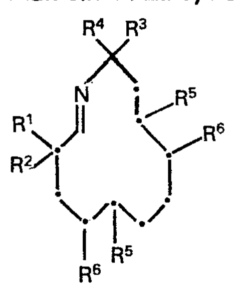 (II)

oder ein 1-Aza-1,5,9-cyclododecatrien der Formel III

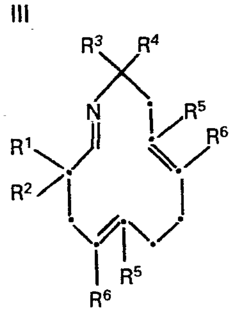 (III)

in wässrigem oder wässrig-organischem Medium in Gegenwart einer anorganischen Säure nach an sich bekannten Verfahren zu Verbindungen der Formel IV

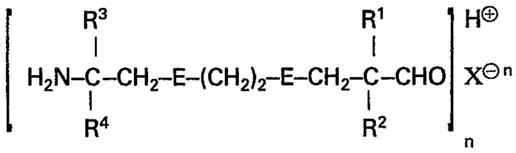

(IV)

umsetzt, wobei in den Formeln II bis IV für $R_1$ bis $R_6$ und E das bei Formel I Angegebenen gilt, X das Anion der anorganischen Säure und n eine der Wertigkeit von X entsprechende ganze Zahl darstellen, und anschliessend die Verbindungen der Formel IV zu den jeweiligen 11-Amino-undecansäuren oder 11-Amino-undeca-4,8-diensäuren der Formel I oxydiert und gegebenenfalls in einer 3. Reaktionsstufe die jeweils erhaltenen Säuren der Formel I nach an sich bekannten Verfahren mit einem Alkohol der Formel $R^7$–OH, in der $R^7$ einen geradkettigen oder verzweigten Alkylrest mit insgesamt 1 bis 18 C-Atomen bedeutet, zu den entsprechenden Estern der Formel I umsetzt.

Geht man bei dem Verfahren von einem 1-Aza-cyclododecan der Formel II aus, so erhält man letztlich die entsprechende 11-Amino-undecansäure bzw. den Ester derselben. Geht man dagegen von einem 1-Aza-1,5,9-cyclododecatrien der Formel III aus, so gelangt man letztlich zu entsprechenden 11-Amino-undeca-4,8-diensäuren bzw. zu den Estern derselben. Sowohl die 11-Amino-undeca-4,8-diensäuren als auch die Ester derselben fallen in Form von Isomeren-Gemischen an.

Die Hydrolyse der Verbindungen der Formeln II oder III gemäss der 1. Stufe des Verfahrens erfolgt ganz analog der in der DE-OS-2 831 299 beschriebenen Verfahrensweise. Sie erfolgt in Gegenwart einer anorganischen Säure in wässrigem oder wässrig-organischem Medium.

Geeignete organische Lösungsmittel sind z.B. Alkohole, besonders solche mit 1–4 C-Atomen, aliphatische Diole, z.B. 1,2-Äthandiol, 1,3-Propandiol und 1,4-Butandiol, und cyclische Äther, z.B. Tetrahydrofuran, Tetrahydropyran und Dioxan. Bevorzugt wird die Hydrolyse in wässrigem Medium vorgenommen.

Als anorganische Säuren können beispielsweise Halogenwasserstoffsäuren, wie HCl und HBr, Schwefelsäure, Phosphorsäure, verdünnte Salpetersäure und Perchlorsäure verwendet werden. Bevorzugt führt man die Hydrolyse in Gegenwart von Schwefelsäure durch.

Die Verbindungen der Formel II oder III und die anorganische Säure werden in mindestens stöchiometrischer Menge eingesetzt. Bevorzugt verwendet man aber einen Überschuss an Säure, z.B. einen bis zu zehnfachen Überschuss und insbesondere einen bis etwa dreifachen Überschuss.

Die Hydrolyse wird im allgemeinen bei Temperaturen zwischen etwa 0 und 60 °C, bevorzugt zwischen etwa 20 und 40 °C, vorgenommen. Die nach der Hydrolyse anfallenden Salze der Formel IV können gewünschtenfalls auf an sich bekannte Weise isoliert werden, z.B. durch Verdampfen der Reaktionslösung. Eine solche Isolierung ist im allgemeinen jedoch nicht erforderlich.

Vor der Oxidationsstufe wird das Reaktionsgemisch jedoch allgemein mindestens 15 Minuten lang mit Wasserdampf behandelt, um aldehydische und andere flüchtige Verunreinigungen zu entfernen. Die 2. Verfahrensstufe der Oxidation erfolgt vorzugsweise in der Weise, dass man das erhaltene Reaktionsgemisch bei Temperaturen zwischen 0° und 200°, vorzugsweise 20° bis 100 °C und einem Druck zwischen 1 bis 100, vorzugsweise 3 bis 20 bar, unter Sauerstoff oder sauerstoffhaltigen Gasen rührt. Es kann auch in Gegenwart von Übergangsmetallsalzen, wie beispielsweise Fe-, Ni-, Cu-, Cr-, Co- und Mn-Salzen, gearbeitet werden, welche die Oxidation katalytisch begünstigen. Spezielle Beispiele für solche Salze sind die folgenden: FE(II)-, FE(III)-, Co(II)-, Co(III)-, Ni(II)-, Mn(II)-, Cu(I)- und Cu(II)-Salze anorganischer oder organischer Säuren, wie HCl, $H_2SO_4$ und Essigsäure. Gut geeignet sind die entsprechenden gut löslichen Acetate, 2-Äthylhexanoate, Acetylacetonate, Phenolate und Stearate.

Das so erhaltene Reaktionsgemisch wird vorzugsweise durch Zugabe von Natronlauge neutralisiert, wobei die jeweilige 11-Amino-undecansäure in Form eines verhältnismässig reinen Produktes ausfällt. Die so erhaltene 11-Amino-undecansäure lässt sich selbstverständlich nach bekannten Verfahren durch Umkristallisieren beispielsweise aus Alkohol-Wasser-Gemischen weiter reinigen. Wird daraus ein erfindungsgemässer Ester der Formel I hergestellt, so lässt sich im allgemeinen die primär ausgefallene und gegebenenfalls mit Wasser gewaschene Säure ohne weitere Reinigungsoperationen verwenden.

Handelt es sich bei dem Produkt, welches bei Neutralisation des Reaktionsgemisches der Oxidationsstufe erhalten wird, um 11-Amino-undeca-4,8-diensäure, so fallen diese im allgemeinen als Isomerengemisch in Form schmieriger Ausscheidungen an.

In den meisten Fällen lässt sich für die Herstellung der erfindungsgemässen 11-Amino-undeca-4,8-diensäureester ebenfalls das ursprüngliche Reaktionsgemisch nach der Oxidationsstufe ohne Abtrennung und Reinigung der Säure direkt weiterverwenden.

Eine Reinigung sowohl der 11-Amino-undecansäuren als auch der 11-Amino-undeca-4,8-diensäuren kann auch in der Weise erfolgen, dass man das jeweilige Rohprodukt zunächst durch Zugabe von wässrigen Säuren oder Basen in Lösung bringt und die so entstandene wässrige Lösung beispielsweise mit einem Äther, wie Diäthyläther, ausschüttelt. Aus der jeweiligen wässrigen Lösung werden nach dieser Reinigung die Aminosäuren dann durch Neutralisation wieder ausgefällt.

Die Veresterung der jeweils erhaltenen 11-Amino-undecansäuren oder 11-Aminoundeca-4,8-diensäuren mit Alkoholen der Formel $R^7$–OH erfolgt nach an sich bekannten Verfahren in Gegenwart von anorganischen Säuren, vorzugsweise Schwefelsäure.

Bei der Herstellung der oben im einzelnen angeführten Vorzugsformen der Verbindungen der Formel I werden die jeweiligen geeigneten Ausgangsprodukte der Formeln II und III und gegebenenfalls der Formel $R^7$-OH eingesetzt, wobei die Reste $R^1$ bis $R^7$ und E jeweils dieselbe Bedeutung wie die der Formel I der herzustellenden speziellen Verbindungen haben.

Die Herstellung der Ausgangsstoffe der Formel III ist u.a. in der DE-OS-2 831 299 bereits ausführlich beschrieben worden. Sie erfolgt wie auch in Helv.Chim.Acta, 61, Fasc. 3, 1122–1124 (1978) beschrieben durch nickel-katalysierte Mischoligomerisation von 2-Aza-1,3-butadienen der Formel V

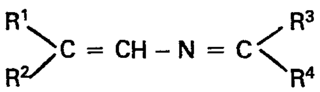

(V)

mit Verbindungen der Formel VI

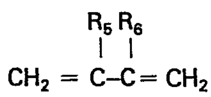

(VI)

wobei $R^1$ bis $R^6$ die unter Formel I angegebene Bedeutung haben. Geeignete Katalysatorsysteme sind z.B. in der deutschen Offenlegungsschrift 2 330 087 beschrieben. Bevorzugt sind Katalysatoren, die in situ durch Reduktion einer kohlenoxidfreien Nickelverbindung, wie Nickelstearat und vor allem Nickelacetylacetonat, mit halogenfreien Metallarylen oder Metallalkylen, z.B. Äthoxy-diäthylaluminium, in Gegenwart eines Alkyl- oder Arylphosphins oder in Gegenwart eines Alkyl- oder Arylphosphits, erhalten werden.

Die obige Umsetzung wird zweckmässig in Gegenwart eines inerten organischen Lösungsmittels, wie n-Hexan, n-Heptan, Benzol, Toluol, Diäthyläther oder Dioxan bei Temperaturen zwischen etwa $-40$ °C und $+150$ °C durchgeführt.

Die 2-Aza-1,3-butadiene der Formel V sind grösstenteils bekannt oder können beispielsweise wie folgt hergestellt werden:

– durch Umsetzung von Aldehyden $R^3$–CHO oder Ketonen 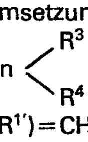 CO mit Alkenylaminen $H_2N–CH_2–C(R^{1'})=CH–R^{2'}$ [ $R^{1'}$ = H oder Alkyl mit 1–8 C-Atomen, $R^{2'}$ = H oder Alkyl C 1–7] und anschliessende Isomerisierung der erhaltenen Verbindungen $R^{2'}$ $–CH=C(R^{1'})–CH_2–N=C(R^3)(R^4)$ in Gegenwart von Katalysatoren, wie $K_2O/Al_2O_3$-Katalysatoren, Alkalimetall- oder Erdalkalimetallalkoholaten [vgl. z.B. B.A. Kazanskii et al., Zhurnal Organicheskoi Khimii, 6, No. 11,2197–99 (1970); Izw. Akad. Nauk SSSR, Ser.Khim., No. 9,2038–2045 (1975) und Tetrahedron, 34, 833–839 (1978)];

– durch Umsetzung von Allylamin oder Methallylamin mit Aldehyden $(R^1)(R^{2''})–CH–CHO$ [$R^{2''}$ wie $R^2$, aber ungleich H] und anschliessende Isomerisierung der erhaltenen Verbindungen $(R^1)(R^{2''})–CH–CH=N–CH_2–C(R)=CH_2$ [R = H oder Methyl) in Gegenwart von Katalysatoren, wie Kalium-tert-butylat;

– durch Umsetzung von Aldehyden $R^1–CH(R^{2''})–CHO$ [$R^{2''}$ gleich wie $R^2$, aber ungleich H) mit Ammoniak (vgl. z.B. US-Patentschrift 2 319 848) und allfällige weitere Umsetzung der dabei erhaltenen Verbindungen $(R^1)(R^{2''})–C=CH–N=CH–CH(R^{2''})(R^1)$ mit geeigneten Ketonen oder Aldehyden (vgl. z.B. US-Patentschrift 3 706 802).

Die Ausgangssubstanzen der Formel II können durch katalytische Hydrierung der 1-Aza-1,5,9-cyclododecatriene der Formel III nach an sich bekannten Methoden hergestellt werden. Diese Hydrierung wird zweckmässig in Gegenwart geeigneter inerter Lösungsmittel und im allgemeinen in geschlossenem System bei einem Druck von etwa 1 bis 200 bar und insbesondere 1 bis 130 bar durchgeführt. Die Hydrierungstemperaturen liegen im allgemeinen zwischen etwa 0 und 150 °C und insbesondere zwischen etwa 25 und 100 °C.

Als Katalysatoren werden vorzugsweise Rhodium/Aluminiumoxid- oder Palladium/Kohle-Katalysatoren eingesetzt.

Die erfindungsgemässen 11-Amino-undecansäureester und 11-Amino-undeca-4,8-diensäureester sind Ausgangsprodukte für die Herstellung von in organischen Lösungsmitteln besonders gut löslichen Salzen anionischer Farbstoffe, vorzugsweise anionischer Azofarbstoffe. Unter Verwendung der erfindungsgemässen Ester lassen sich insbesondere die sehr günstigen Azofarbstoffsulfonsäuresalze der Formel VII

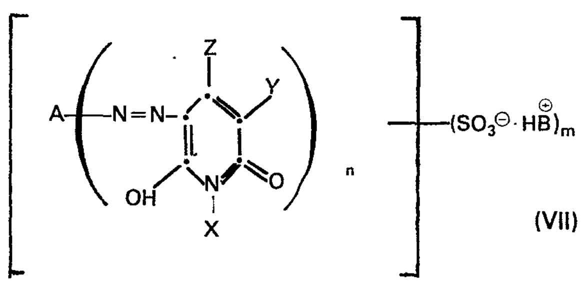

(VII)

oder einer dazu tautomeren Formel herstellen, worin A einen carbocyclischen oder heterocyclischen aromatischen Rest, B den jeweiligen 11-Amino-undecansäureester oder 11-Amino-undeca-4,8-diensäureester der Formel I, X ein H-Atom oder eine gegebenenfalls substituierte Alkylgruppe, eine Cycloalkyl-, Aralkyl- oder Arylgruppe, Y ein H- oder Halogenatom oder eine elektronenanziehende Gruppe wie z.B. eine Nitro-, Cyan-, Acyl-, Sulfonsäure-, Arylsulfonyl-, Alkoxycarbonyl- oder eine gegebenenfalls substituierte Alkyl-, Sulfamoyl- oder Carbamoylgruppe, Z eine gegebenenfalls substituierte Alkylgruppe oder einen Arylrest, m und n die Zahl 1 oder 2 bedeuten.

Sofern n für die Zahl 1 steht, bedeutet A einen einwertigen, vorzugsweise carbocyclischen aromatischen Rest, beispielsweise einen Naphthalinrest, insbesondere aber einen gegebenenfalls substituierten Phenylrest. Steht n für die Zahl 2, bedeutet A also einen zweiwertigen Rest, so ist dies vorzugsweise ein Diphenyl-, Diphenyläther- oder Diphenylsulfonrest. Steht der Substituent X

für eine Alkylgruppe, so handelt es sich vorzugsweise um eine $C_1$–$C_{18}$-Alkylgruppe, die beispielsweise durch Hydroxygruppen, Alkoxygruppen mit 1–8 C-Atomen oder gegebenenfalls durch Chlor, Methyl, oder $-SO_3H$ substituierte Phenylgruppen substituiert sein kann. Wenn n = 1 bedeutet, so kann die Alkylgruppe auch einen Rest der Formel

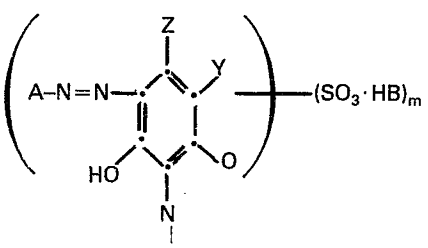

enthalten. Steht X für Cycloalkyl, so handelt es sich vornehmlich um Cyclohexyl; steht X für Aralkyl, so handelt es sich vornehmlich um eine Phenylalkylgruppe mit 1–4 C-Atomen im Alkylrest und bedeutet X Aryl, dann vorzugsweise gegebenenfalls durch Chlor, Methyl oder $-SO_3H$ substituiertes Phenyl. Steht Y für eine Alkylgruppe, so handelt es sich vorzugsweise um eine $C_1$–$C_6$-Alkylgruppe, die als Substituenten beispielsweise eine Sulfonsäuregruppe oder eine Alkanoylaminogruppe mit 2–7 C-Atomen aufweisen kann; steht Y für eine Acylgruppe, so handelt es sich vorzugsweise um eine Alkanoylgruppe mit 2–7 C-Atomen oder eine Benzoylgruppe. Steht Y für Carbamoyl, oder Sulfamoyl, so können dieselben unsubstituiert oder durch eine oder zwei Alkylgruppen mit 1–6 C-Atomen oder einen gegebenenfalls Chloratome oder Methylgruppen enthaltenden Phenylrest substituiert sein. Steht Y für eine Alkoxycarbonylgruppe, so handelt es sich vorzugsweise um eine solche mit 2–7 C-Atomen, ist Y eine Arylsulfonylgruppe, dann vorzugsweise eine Phenylsulfonylgruppe. Steht Z für eine Alkylgruppe, so handelt es sich vorzugsweise um eine solche mit 1–6 C-Atomen, die gegebenenfalls eine Sulfonsäuregruppe oder einen Phenylrest aufweisen kann. Bedeutet Z einen Arylrest, so handelt es sich vorzugsweise um einen Phenylrest, der durch Chlor, Methyl oder Alkoxy mit 1–6 C-Atomen substituiert sein kann.

Zu den Farbsalzen gelangt man, wenn man die entsprechende Azofarbstoffmono- oder disulfonsäure mit 1 bzw. 2 Mol des Aminoesters B umsetzt.

Die Azofarbstoffsulfonsäuren stellen bekannte Verbindungen dar, die beispielsweise in den folgenden Veröffentlichungen beschrieben sind: DE-OS-1 924 570, 2 004 487, 2 050 901, 2 134 453, 2 150 817, 2 237 006, 2 533 723, 1 930 491, 2 004 488, 2 115 449, 2 141 449, 2 162 612, 2 238 795, 2 545 828, 1 956 142, 2 033 281, 2 123 061, 2 141 453, 2 216 206, 2 349 709, 2 701 290, GP-PS-1 296 857, 1 331 261, 1 345 864.

Man erhält die Azofarbstoffsulfonsäuren durch Kuppeln eines diazotierten oder tetrazotierten carbocyclischen oder heterocyclischen aromatischen Amins mit einem Pyridon der Formel

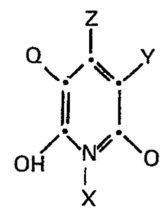

oder einer dazu tautomeren Formel, worin Q ein H-Atom oder einen leicht abspaltbaren Rest, beispielsweise die Carbamoylgruppe bedeutet, X, Y und Z die angegebene Bedeutung haben, wobei die Komponenten so auszuwählen sind, dass der fertige Azofarbstoff mindestens eine Sulfonsäuregruppe enthält.

Die Salzbildung führt man zweckmässig in der Weise durch, dass man eine Lösung oder Suspension eines Alkalisalzes der Farbstoffsulfonsäure mit der wässrigen Lösung eines wasserlöslichen Salzes des jeweiligen 11-Amino-undecansäureesters oder 11-Amino-undeca-4,8-diensäureesters, vorzugsweise eines solchen mit einer niederen Fettsäure, insbesondere der Ameisensäure oder Essigsäure umsetzt. Man arbeitet mit Vorteil bei Temperaturen zwischen 40–80 °C und einem pH-Wert unterhalb 7.

Da die Farbsalze im wässrigen Reaktionsmedium unlöslich sind, können sie durch Abfiltrieren isoliert werden.

Man kann die Umsetzung aber auch in organischen Lösungsmitteln allein oder in Gemischen mit Wasser durchführen.

Die so erhältlichen Farbsalze weisen eine ausgezeichnete Alkohollöslichkeit auf, insbesondere in niederen Alkanolen, wie Methanol, Äthanol, n-Propanol oder iso-Propanol, in Alkylenglykolmonoalkyläthern, z.B. in Äthylenglykol-monomethyl- oder -äthyläther, in Alkylenglykolen, wie in Propylenglykolen, oder in araliphatischen Alkoholen, wie in Benzylalkohol, oder in Gemischen derartiger Alkohole, ferner in niederen aliphatischen Ketonen, z.B. Aceton, Methyläthylketon, Methylisobutylketon oder auch in Cyclohexanon, ferner in Carbonsäureestern, z.B. Methylacetat, Äthylacetat, Butylacetat oder Glykolmonoacetat, sowie in halogenierten Kohlenwasserstoffen, vorzugsweise niederen aliphatischen Kohlenwasserstoffen, wie Chloroform, Methylenchlorid, Äthylenchlorid oder Kohlenstofftetrachlorid.

Wegen ihrer guten Löslichkeit eignen sich die beschriebenen Farbsalze zum Färben von Cellulose-2 1/2-acetat in der Spinnmasse und für die Spinnfärbung von Cellulosetriacetat. Das spinngefärbte Fasermaterial zeichnet sich durch Reinheit und Stärke des Farbtons, durch einwandfreie Verteilung des Farbkörpers und durch sehr gute Echtheiten wie beispielsweise hohe Wasch-, Wasser-, Bleich-, Überfärbe-, Trockenwasch-, Reib-, Bügel-, Trockenhitze- und Lichtechtheit aus.

Infolge ihrer guten Löslichkeit in Alkoholen, Estern sowie deren Mischungen, sind die neuen Farbsalze besonders geeignet zum Färben von filmbildenden Polymeren.

Als alkoholische und/oder esterhaltige Lösungen filmbildender Polymerer sind hier insbesondere solche flüssige Lacke zu versehen, die sich zur Verwendung in Druckfarben für den Flexodruck eignen. Als Polymere enthalten diese Lösungen beispielsweise Naturharze, wie Schellack oder Manilakopal; oder Cellulosederivate, beispielsweise Celluloseäther, wie Äthylcellulose oder Celluloseester, wie Nitrocellulose, ferner Maleinatharze oder Phenol-Formaldehydharze, Polyamidharze, Formaldehyd-Harnstoff- und Formaldehyd-Melamin-Kondensate, Keton-Formaldehyd-Kondensate, Polyvinylacetate oder Polyacrylsäureharze, z.B. Polybutylacrylatharz oder deren Gemische; ferner Polykondensationsprodukte mehrwertiger Alkohole, wie Glycerin oder Pentaerythrit, mit mehrbasischen Säuren, wie Maleinsäure oder Phthalsäure allein oder in Kombination mit ungesättigten Fettsäuren wie jenen des Lein- und Ricinusöls.

Diese Lösungen filmbildender Polymerer mit einem Gehalt an diesen Farbsalzen eignen sich beispielsweise zum Bedrucken verschiedenartiger Stoffe, wie von Metall-, z.B. Aluminiumfolien, von Papier, Glas, Kunstharzfolien und -filmen und dergleichen.

Die Lösungen filmbildender Polymerer eignen sich weiter zum Lackieren verschiedenster Oberflächen, z.B. von Metallteilen, Formteilen aus Kunststoffen oder Holzplatten. Sie sind lagerbeständig und ergeben auf den genannten Materialien egale, farbstarke und wasserfeste Überzüge.

Herstellungsbeispiele
A) Verbindungen der Formel I

Beispiel 1

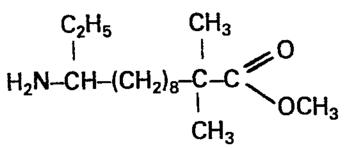

a) 975 g (4,37 Mol) 3,3-Dimethyl-12-äthyl-1-aza-cyclododecen (hergestellt gemäss Beispiel β) werden innerhalb von ca. 35 Minuten, unter Rühren, zu einer Lösung von 450 g (4,6 Mol) Schwefelsäure in 600 g Wasser eingetropft. Die klare, leicht gelbliche Lösung wird dann während 20 Minuten mit Wasserdampf behandelt, um eventuelle aldehydische Verunreinigungen zu entfernen. Anschliessend wird die wässrige Lösung in einem Autoklaven 3 Stunden bei 50 °C unter einem Sauerstoffdruck von 20 bar gerührt. Beim Neutralisieren der sauren Reaktionsmischung mit Natronlauge erhält man 820 g (3,2 Mol) 2,2-Dimethyl-11-äthyl-11-amino-undecansäure, entsprechend einer Ausbeute von 73% d.Th.;

Fp. 164–165 °C;

Analyse für $C_{15}H_{31}NO_2$ (Molgewicht 257,42)
berechnet:
C 69,99% H 12,14% N 5,44% O 12,43%
gefunden:
C 70,03% H 12,10% N 5,54% –

MS-Spektrum: Molekül-Peak 257, Bruchstückmassen 228, 182, 140, 58.

b) 51,4 g (0,2 Mol) 2,2-Dimethyl-11-äthyl-11-amino-undecansäure werden zusammen mit 200 ml Methanol und 22 g (0,224 Mol) Schwefelsäure während 3 Stunden unter Rückfluss gekocht. Man destilliert dann das überschüssige Methanol ab, versetzt den Rückstand mit ca. 200 ml Wasser und bringt die Reaktionslösung mit wässriger Natronlauge auf schwach alkalisch (pH 8–10), wobei sich der Aminosäureester als obere organische Phase abscheidet. Bei der anschliessenden Destillation erhält man 48,5 g (0,179 Mol) 2,2-Dimethyl-11-äthyl-11-aminoundecansäuremethylester, entsprechend einer Ausbeute von 89,5% d.Th.;

Sdp. 78–80 °C/0,05 Torr, $n_D^{20}$ = 1,4494.

Analyse für $C_{16}H_{33}NO_2$ (Molgewicht 271,45)
berechnet:
C 70,80% H 12,25% N 5,16% O 11,79%
gefunden:
C 70,54% H 12,13% N 5,10% O 11,70%

MS-Spektrum: Molekül-Peak 271, Bruchstückmassen 242, 212, 182, 102, 58

$^1$H–NMR-Spektrum τ (ppm): 6,43(s), 7,48(m), 8,75(s), 8,89(s) und 8,93(s), 9,13(t) im Verhältnis 3:1:18:8:3

IR-Sektrum (flüssig): ν (C=O) – 1745 $cm^{-1}$

Beispiel 2

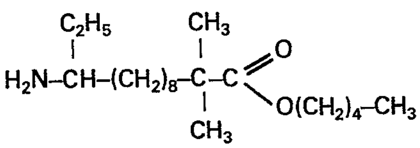

Es wird wie in Beispiel 1b) beschrieben vorgegangen, jedoch unter Verwendung von 200 ml Pentanol anstelle von 200 ml Methanol. Nach der Destillation erhält man 60,5 g (0,185 Mol) 2,2-Dimethyl-11-äthyl-11-aminoundecansäurepentylester, entsprechend einer Ausbeute von 92,5% d.Th.; Sdp. 126–128 °C/0,02 Torr; $n_D^{20}$ = 1,4494;

Analyse für $C_{20}H_{41}NO_2$ (Molgewicht 327)
berechnet:
C 73,40% H 12,55% N 4,28% O 9,78%
gefunden:
C 73,43% H 12,55% N 4,51% O 9,77%

MS-Spektrum: Molekül-Peak 327, Bruchstückmassen 298, 240, 212, 182, 170;

$^1$H–NMR-Spektrum τ (ppm): 5,96(t), 7,38(m), 8,2–8,7(m) und 8,80(s), 9,05(t) im Verhältnis 2:1:35:3

IR-Spektrum (flüssig): ν (C=O) – 1750 $cm^{-1}$.

Beispiel 3

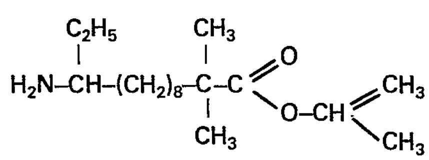

Es wird wie in Beispiel 1b) beschrieben vorgegangen, jedoch mit folgenden Abänderungen an dem Verfahren. Es werden 150 ml Isopropanol anstelle von 200 ml Methanol eingesetzt. Anstelle von 22 g (0,224 Mol) Schwefelsäure wird ein Überschuss an gasförmigem HCl verwendet. Nach einer Reaktionszeit von 8 Stunden bei Rückflusstemperatur erhält man bei der anschliessenden Destillation 42 g (0,141 Mol) 2,2-Dimethyl-11-äthyl-11-aminoundecansäureisopropylester, entsprechend einer Ausbeute von 70,5% d.TH.; Sdp. 95 °C/0,01 Torr; $n_D^{20}$ = 1,4469;

Analyse für $C_{18}H_{37}NO_2$ (Molgewicht 299,50)
berechnet:
C 72,10% H 12,45% N 4,68% O 10,68%
gefunden:
C 72,13% H 12,86% N 4,96% O 9,34%

MS-Spektrum: Molekül-Peak 299, Bruchstückmassen 270, 240, 212, 182, 170, 69;

$^1$H–NMR-Spektrum τ (ppm): 5,06(sept), 7,4(m), 8,4–8,75(m) und 8,80(s) und 8,85(s), 9,08(t) im Verhältnis 1:1:32:3;

IR-Spektrum (flüssig): ν (C=O) – 1745 $cm^{-1}$

Beispiel 4

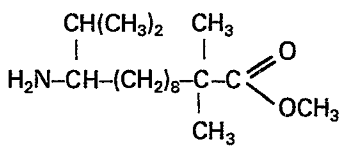

a) Es wird wie in Beispiel 1a) beschrieben vorgegangen, jedoch unter Verwendung von 500 g (2,11 Mol) 3,3-Dimethyl-12-isopropyl-1-aza-cyclododecen (hergestellt gemäss Beispiel α), 250 g (2,55 Mol) Schwefelsäure und 1 kg Wasser. Nach einer Reaktionszeit von 6 Stunden bei 50 °C unter einem Sauerstoffdruck von 5 bar erhält man 542 g (2 Mol) 2,2-Dimethyl-11-isopropyl-11-aminoundecansäure, entsprechend einer Ausbeute von 94,7% d.Th.; Fp. 156–159 °C;

Analyse für $C_{16}H_{33}NO_2$ (Molgewicht 271,45)
berechnet:
C 70,80% H 12,25% N 5,16% O 11,79%
gefunden:
C 70,86% H 12,43% N 5,16% O 11,66%

MS-Spektrum: Molekül-Peak 271, Bruchstückmassen 256, 228, 140, 72.

b) Es wird wie in Beispiel 1b) beschrieben vorgegangen, jedoch unter Verwendung von 54,2 g (0,2 Mol) 2,2-Dimethyl-11-isopropyl-11-aminoundecansäure. Nach der Destillation erhält man 51 g (0,179 Mol) 2,2-Dimethyl-11-isopropyl-11-aminoundecansäuremethylester, entsprechend einer Ausbeute von 89,5% d.Th.; Sdp. 106–107 °C/0,03 Torr; $n_D^{20}$ = 1,4511;

Analyse für $C_{17}H_{35}NO_2$ (Molgewicht 285,47)
berechnet:
C 71,53% H 12,36% N 4,91% O 11,21%
gefunden:
C 71,69% H 12,52% N 4,99% O 11,23%

MS-Spektrum: Molekül-Peak 285, Bruchstückmassen 270, 254, 285, 182, 140, 102, 72;

$^1$H–NMR-Spektrum τ (ppm): 6,33(s), 7,5(m), 8,2–8,7(m), 8,82(s), 8,97(s), 9,12(dd) im Verhältnis 3:1:17:6:2:6;

IR-Spektrum (flüssig): ν (C=O) – 1745 $cm^{-1}$.

Beispiel 5

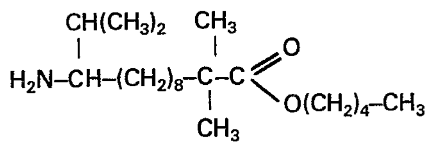

Es wird wie in Beispiel 1b) beschrieben vorgegangen, jedoch unter Verwendung von 52,3 g (0,193 Mol) 2,2-Dimethyl-11-isopropyl-11-aminoundecansäure und 200 ml n-Pentanol. Nach der Destillation erhält man 60,5 g (0,178 Mol) 2,2-Dimethyl-11-isopropyl-11-amino-undecansäurepentylester, entsprechend einer Ausbeute von 92,2% d.Th.; Sdp. 138–139 °C/0,01 Torr; $n_D^{20}$= 1,4505;

Analyse für $C_{21}H_{43}NO_2$ (Molgewicht 341)
berechnet:
C 73,80% H 12,65% N 4,10% O 9,38%
gefunden:
C 73,99% H 12,77% N 4,16% O 9,36%

MS-Spektrum: Molekül-Peak 341, Bruchstückmassen 298, 254, 226, 182, 140, 72;

$^1$H–NMR-Spektrum τ (ppm): 5,98(t), 7,5(m), 8,3–8,7(m), 8,80(s), 8,89(s), 9,09(dt) im Verhältnis 2:1:26:6:2:6;

IR-Spektrum (flüssig): ν (C=O) – 1750 $cm^{-1}$.

Beispiel 6

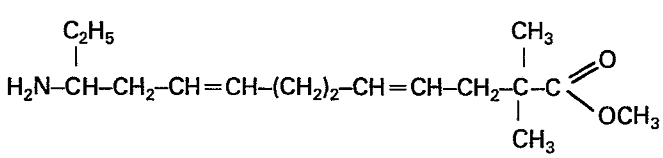

Es wird wie in Beispiel 1a) beschrieben vorgegangen, jedoch unter Verwendung von 55 g (0,251 Mol) 3,3-Dimethyl-12-äthyl-1-aza-1,5,9-cyclododecatrien, 27 g (0,275 Mol) Schwefelsäure und 500 g Wasser. Die anfallende schmierige Aminosäure wird dann sofort, wie in Beispiel 1b) beschrieben, in Gegenwart von 100 ml Methanol weiter umgesetzt. Nach der Destillation erhält man 10,5 g (0,0394 Mol) 2,2-Dimethyl-11-äthyl-11-amino-undeca-4,8-diensäure-methylester, entsprechend einer Ausbeute von 15,7% d.Th.; Sdp. 106–108 °C/0,3 bar; $n_D^{20}$ = 1,4754;

Analyse für $C_{16}H_{29}NO_2$ (Molgewicht 267,41)
berechnet:

C 71,87% H 10,93% N 5,24% O 11,97%
gefunden:
C 71,55% H 10,93% N 5,64% O 11,94%

MS-Spektrum: Molekül-Peak 267, Bruchstückmassen 238, 236, 208, 166, 140, 95;

$^1$H–NMR-Spektrum τ (ppm): 4,6(m), 6,36(s), 7,35(m), 7,7–8,0(m), 8,4(s) u. 8,57(m), 8,82(s), 9,06(t) im Verhältnis 4:3:1:8:4:6:3.

Beispiel 7

Es wird wie in Beispiel 1b) beschrieben vorgegangen, jedoch unter Verwendung von 50 g (0,185 Mol) 2,2-Dimethyl-11-isopropyl-11-aminoundecansäure und 100 ml Isopropanol. Nach der Destillation erhält man 44 g (0,141 Mol) 2,2-Dimethyl-11-isopropyl-11-amino-undecansäureisopropylester, entsprechend einer Ausbeute von 76% d.Th.; Sdp. 118–120 °C/0,03 Torr; $n_D^{20}$ = 1,4481;

Analyse für $C_{19}H_{39}NO_2$ (Molgewicht 313,53)
berechnet:
C 72,79% H 12,54% N 4,47% O 10,21%
gefunden:
C 72,46% H 12,69% N 4,71% O 9,56%

MS-Spektrum: Molekül-Peak 313, Bruchstückmassen 298, 270, 254, 226, 182, 72

$^1$H–NMR- Spektrum τ (ppm): 5,05 (sep), 7,5 (m), 8,2–9,0 (m) und 9,1 (dd) im Verhältnis 1:1:31:6.

B) Verbindungen der Formel II

Beispiel α

466,8 g (2 Mol) 3,3-Dimethyl-12-isopropyl-1-aza-1,5,9-cyclododecatrien werden in 4 Liter Cyclohexan gelöst und bei 20–25 °C und einem Anfangsdruck von 100 bar in Gegenwart von 50 g Rhodium/Aluminiumoxid während 4 Stunden in einem Stahlautoklaven hydriert. Nach dem Abdestillieren des Lösunqsmittels erhält man als Hauptfraktion 425 g (1,79 Mol) 3,3-Dimethyl-12-isopropyl-1-aza-cyclododecen; Sdp. 92–94 °C/4 Pa; $n_D^{20}$ = 1,4706.

Beispiel β

Es wird wie in Beispiel α beschrieben vorgegangen, jedoch unter Verwendung von 438 g (2 Mol) 3,3-Dimethyl-12-äthyl-1-aza-1,5,9-cyclododecatrien. Nach der Destillation erhält man 412 g (1,85 Mol) 3,3-Dimethyl-12-äthyl-1-aza-cyclododecen; Sdp. 61–63 °C/4 Pa; $n_D^{20}$ = 1,4721.

Beispiel γ

Es wird wie in Beispiel α beschrieben vorgegangen, jedoch unter Verwendung von 253,4 g (1 Mol) 3-Methyl-12-phenyl-1-aza-1,5,9-cyclododecatrien. Nach der Destillation erhält man 243 g (0,945 Mol) 3-Methyl-12-phenyl-1-aza-cyclododecen; Sdp. 110–112 °C/0,03 Torr.

Beispiel 8

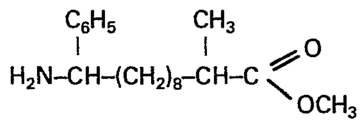

Es wird wie in Beispiel 1 a) vorgegangen, jedoch unter Verwendung von 65 g (0,253 Mol) 3-Methyl-12-phenyl-1-aza-cyclododecen. Nach beendeter Oxydation wird, ohne Isolierung der Aminosäure, wie in Beispiel 1 b) beschrieben vorgegangen (unter Verwendung von 500 ml Methanol). Nach der Destillation erhält man 27 g (0,0885 Mol) 2-Methyl-11-phenyl-11-amino-undecansäuremethylester, entsprechend einer Ausbeute von 35% d.Th; Sdp. 135 °C/0,02 Torr; $n_D^{20}$ = 1,5000

Analyse für $C_{19}H_{31}NO_2$ (Molgewicht 305,46)
berechnet:
C 74,71% H 10,23% N 4,58% O 10,47%
gefunden:
C 75,43% H 10,62% N 4,68% O 9,90%

MS-Spektrum: Molekül-Peak 305, Bruchstückmassen 274, 200, 106

$^1$H–NMR-Spektrum τ (ppm): 2,71(s), 6,14(t), 6,36(s), 7,6(m), 8,2–8,75 (m) und 8,84(d) im Verhältnis 5:1:3:1:18:3.

Anwendungsbeispiele

Beispiel I

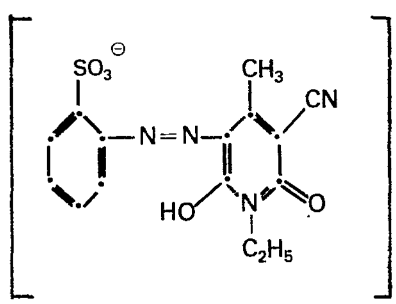
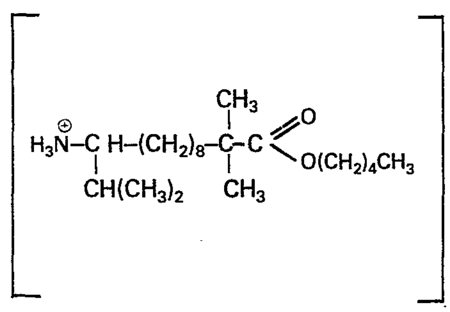

19,2 g (0,05 Mol) des Monoazofarbstoffes 2-Aminobenzolsulfonsäure → 1-Äthyl-2-hydroxy-4-methyl-5-cyan-pyridon-6 (Na-Salz) werden in 500 ml Wasser angeschlämmt und tropfenweise mit der Mischung von 17,1 g (0,05 Mol) 2,2-Dimethyl-11-isopropyl-11-amino-undecansäure-pentylester (siehe Beispiel 5), 4 ml 85%iger Ameisensäure und 80 ml Wasser versetzt. Man rührt die Suspension einige Stunden bei 45–50 °C, filtriert sie,

wäscht das Filtergut zuerst mit verdünnter Ameisensäure, dann mit Wasser und trocknet es bei 60 °C im Vakuum. Der so isolierte Solventfarbstoff obenstehender Formel wiegt 35,7 g. Er ist in Äthanol, Aceton, Äthylacetat und Äthylenglykolmonoäthyläther sehr gut löslich.

Ebenfalls wertvolle gelbe Solventfarbstoffe erhält man bei analoger Arbeitsweise, wenn man anstelle der 0,05 Mol 2,2-Dimethyl-11-isopropyl-11-amino-undecansäure-pentylester die äquimolekulare Menge eines der gemäss Beispiel 1–4 und 6–8 hergestellten Amine einsetzt.

Beispiel II

48,6 g (0,1 Mol) des Natriumsalzes des durch Kuppeln von Anilin-2,4-disulfonsäure auf 1-Äthyl-2-hydroxy-4-methyl-5-cyanpyridon-6 erhaltenen Monoazofarbstoffs werden in 300 ml Wasser und 300 ml Methylisobutylketon eingerührt. Zur Mischung lässt man 68,4 (0,2 Mol) 2,2-Dimethyl-11-isopropyl-11-amino-undecansäure-pentylester und Ameisensäure bis zu einem pH-Wert von 3,5–4 tropfen.

Man rührt das Ganze während 1 Std. bei 40–45 °C, trennt die beiden Phasen und wäscht die organische Phase mit 250 ml Wasser. Das durch Abdestillieren des Lösungsmittels erhältliche gelbe Farbstoffsalz wiegt nach dem Trocknen im Vakuum bei erhöhter Temperatur 88 g. Es ist in den gebräuchlichen Lösungsmitteln der Druckfarben- und Lackindustrie, wie z.B. in Aceton, Methanol, Äthanol, Isopropanol, n-Butanol, Benzylalkohol, Äthylenglykolmonomethyl-und monoäthyläther, sowie in Lösungsmittelgemischen wie Äthanol/Toluol 70:30, Äthanol/Äthylenglykolmonoäthyläther 85:15, Äthanol/Äthylacetat 50:50; Methylenchlorid/Methanol 9:1 sehr gut löslich. Die damit erhaltenen Druckfarben, gefärbten Lacke und Acetatfasern zeichnen sich durch einen reinen, gelben Farbton aus.

Beispiel III

5 g des nach Beispiel I erhaltenen Farbsalzes werden in 95 g eines Nitrocelluloselackes, hergestellt aus 15 g alkohollöslicher niedrigviskoser Nitrocellulose mit ca. 18% Dibutylphthalat, 10 g Äthylenglykolmonoäthyläther, 20 g Äthylacetat und 50 g 94%igem Äthanol, eingetragen. Die Mischung wird bis zur gleichmässigen Verteilung des Farbkörpers gerührt. Anschliessend wird der Lack mit einem Filmziehgerät (Handcoater der Fa. RK Chemical Co. Ltd., Royston, GB) in einer Nassfilmdicke von ca. 12 µm auf Opalinpapier oder auf eine Aluminium-Kaschierfolie aufgetragen. Nach dem Trocknen liegt eine haftfeste, gleichmässige, kräftig gelbe Lackierung vor, die gegenüber Belichtung sowie Behandlung mit Wasser und Butter hohe Beständigkeiten aufweist.

Vergleichsbeispiel IV

Es wird wie in Beispiel I beschrieben vorgegangen, jedoch unter Verwendung von 12,2 g (0,05 Mol) 11-Aminoundecansäure-isopropylester. Dabei fallen 29,8 g eines gelben Farbsalzes an, das in den gebräuchlichen Lösungsmitteln nur sehr wenig löslich ist. Im Gegensatz zu den Farbsalzen aus verzweigten Aminoundecansäureestern ist es nicht als Solventfarbstoff einsetzbar.

**Patentansprüche**

1. Verbindungen der Formel I

$$H_2N-\overset{R^3}{\underset{R^4}{C}}-CH_2-E-(CH_2)_2-E-CH_2-\overset{R^1}{\underset{R^2}{C}}-CO\cdot OR^7 \qquad (I)$$

worin $R^1$ und $R^3$ unabhängig voneinander Wasserstoff oder einen geradkettigen oder verzweigten Alkylrest mit 1–8 C-Atomen, $R_3$ ausserdem einen Phenylrest, $R^2$ eine geradkettigen oder verzweigten Alkylrest mit 1–8 C-Atomen und $R^4$ einen geradkettigen oder verzweigten Alkylrest mit 1–18 C-Atomen darstellen oder $R^1$ und $R^2$ und/oder $R^3$ und $R^4$ zusammen mit dem Bindungs-C-Atom einen cycloaliphatischen Ring mit 4–8 C-Atomen bilden, und worin E jeweils einen der Reste $-\overset{R^5}{C}H-\overset{R^6}{C}H-$ und $-\overset{R^5}{C}=\overset{R^6}{C}-$ bedeutetn, in denen $R^5$ und $R^6$ unabhängig voneinander für Wasserstoff oder Alkyl mit 1–4 C-Atomen stehen und $R^7$ Wasserstoff oder einen geradkettigen oder verzweigten Alkylrest mit insgesamt 1 bis 18 C-Atomen darstellt.

2. 11-Amino-undecansäuren nach Anspruch 1, dadurch gekennzeichnet, dass in Formel I $R^7$ –H und E den Rest $-\overset{R^5}{C}H-\overset{R^6}{C}H-$ bedeuten.

3. 11-Amino-undecansäureester nach Anspruch 1, dadurch gekennzeichnet, dass in Formel I $R^7$ einen geradkettigen oder verzweigten Alkylrest mit insgesamt 1 bis 18, vorzugsweise 1 bis 8, C-Atomen und E den Rest $-\overset{R^5}{C}H-\overset{R^6}{C}H-$ bedeuten.

4. 11-Amino-undeca-4,8-diensäureester nach Anspruch 1, dadurch gekennzeichnet, dass in Formel I $R^7$ einen geradkettigen oder verzweigten Alkylrest mit insgesamt 1 bis 18, vorzugsweise 1 bis 8, C-Atomen und E den Rest $-\overset{R^5}{C}=\overset{R^6}{C}-$ bedeuten.

5. Verbindungen der Formel I nach Anspruch 1, worin $R^5$ und $R^6$ je Wasserstoff, $R^1$ und $R^3$ unabhängig voneinander Wasserstoff oder Alkyl mit 1–5 C-Atomen, $R^2$ Alkyl mit 1–5 C-Atomen und $R^4$ Alkyl mit 1–7 C-Atomen darstellen, oder worin $R^3$, $R^5$ und $R^6$ je Wasserstoff, $R^1$ und $R^2$ zusammen mit dem Bindungs-C-Atom Cyclopentyl oder Cyclohexyl und $R^4$ Alkyl mit 1–7 C-Atomen bedeuten.

6. Verbindungen der Formel I nach Anspruch 1, worin $R^3$, $R^5$ und $R^6$ je Wasserstoff, $R^1$ und $R^2$ unabhängig voneinander Alkyl mit 1–4 C-Atomen oder zusammen mit dem Bindungs-C-Atom Cyclohexyl und $R^4$ Alkyl mit 1–7 C-Atomen darstellen.

7. Verbindungen der Formel I nach Anspruch 1, worin $R^3$ Wasserstoff und $R^4$ den Rest der Formel

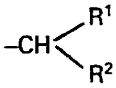 bedeuten.

8. Eine Verbindung der Formel I nach Anspruch 1, worin $R^1$ und $R^2$ je $-CH_3$, $R^3$ $-H$, $R^4$ $-CH(CH_3)_2$, E $-CH_2CH_2-$ und $R^7$ n-Pentyl bedeuten.

9. Verfahren zur Herstellung von Verbindungen der Formel I nach Anspruch 1, dadurch gekennzeichnet, dass man ein 1-Aza-cyclododecen der Formel II

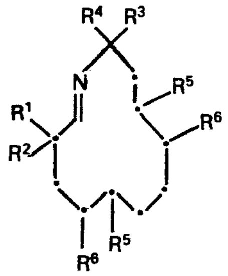

(II)

oder ein 1-Aza-1,5,9-cyclododecatrien der Formel III

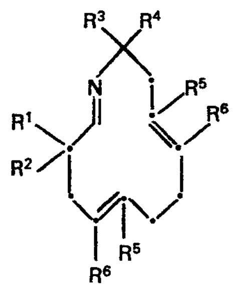

(III)

in wässrigem oder wässrig-organischem Medium in Gegenwart einer anorganischen Säure nach an sich bekannten Verfahren zu Verbindungen der Formel IV

$$\left[ H_2N-\underset{R^4}{\overset{R^3}{C}}-CH_2-E-(CH_2)_2-E-CH_2-\underset{R^2}{\overset{R^1}{C}}-CHO \;\middle|\; \begin{matrix} H^{\oplus} \\ X^{\ominus n} \end{matrix} \right]_n \quad (IV)$$

umsetzt, wobei in den Formeln II bis IV für $R_1$ bis $R_6$ und E das im Anspruch 1 Angegebene gilt, X das Anion der anorganischen Säure und n eine der Wertigkeit von X entsprechende ganze Zahl darstellen, und anschliessend die Verbindungen der Formel IV zu den jeweiligen 11-Amino-undecansäuren oder 11-Amino-undeca-4,8-diensäuren der Formel I oxydiert und gegebenenfalls in einer 3. Reaktionsstufe die jeweils erhaltenen Säuren der Formel I nach an sich bekannten Verfahren mit einem Alkohol der Formel $R^7$–OH, in der $R^7$ einen geradkettigen oder verzweigten Alkylrest mit insgesamt 1 bis 18 C-Atomen bedeutet, zu den entsprechenden Estern der Formel I umsetzt.

10. Verfahren nach Anspruch 9, dadurch gekennzeichnet, dass man die Umsetzung einer Verbindung der Formel II oder III zu Verbindungen der Formel IV in wässrigem Medium in Gegenwart von Schwefelsäure durchführt.

11. Verfahren nach Anspruch 9, dadurch gekennzeichnet, dass man die Verbindungen der Formel IV durch Behandlung mit Sauerstoff unter Überdruck zu den jeweiligen 11-Amino-undecansäuren oder 11-Amino-undeca-4,8-diensäuren der Formel I oxydiert.

12. Verfahren nach Anspruch 11, dadurch gekennzeichnet, dass man die Oxydation bei einem Druck von 1 bis 100, vorzugsweise 3 bis 20, bar und bei einer Temperatur von 0 bis 200 °, vorzugsweise 20 bis 100 °C, durchführt.

13. Verfahren nach Anspruch 9 zur Herstellung von Verbindungen nach den Ansprüchen 2 bis 8, dadurch gekennzeichnet, dass man ein 1-Aza-cyclododecen der Formel II oder ein 1-Aza-1,5,9-cyclododecatrien der Formel III und gegebenenfalls einen Alkohol der Formel $R^7$–OH einsetzt, wobei in den Formeln II, III und $R^7$–OH die Reste $R^1$ bis $R^7$ die jeweilige Bedeutung wie in den Ansprüchen 2 bis 8 haben.

**Revendications**

1. Composés répondant à la formule (I)

$$H_2N-\underset{R^4}{\overset{R^3}{C}}-CH_2-E-(CH_2)_2-E-CH_2-\underset{R^2}{\overset{R^1}{C}}-CO\cdot OR^7 \quad (I)$$

dans laquelle

$R^1$ et $R^3$ représentent chacun, indépendamment l'un de l'autre, l'hydrogène ou un radical alkyle linéaire ou ramifié qui contient de 1 à 8 atomes de carbone, $R^3$ pouvant en outre représenter un radical phényle, $R^2$ représente un radical alkyle linéaire ou ramifié contenant de 1 à 8 atomes de carbone, et

$R^4$ représente un radical alkyle linéaire ou ramifié contenant de 1 à 18 atomes de carbone,

ou

$R^1$ et $R^2$, et/ou $R^3$ et $R^4$, forment ensemble et avec l'atome de carbone qui les unit un noyau cycloaliphatique contenant de 4 à 8 atomes de carbone, les E représentent chacun l'un des radicaux: $-\overset{R^5}{CH}-\overset{R^6}{CH}-$ et $-\overset{R^5}{C}=\overset{R^6}{C}-$ dans lesquels $R^5$ et $R^6$ représentent chacun, indépendamment l'un de l'autre, l'hydrogène ou un alkyle en $C_1$–$C_4$, et

$R^7$ représente l'hydrogène ou un radical alkyle linéaire ou ramifié qui contient au total de 1 à 18 atomes de carbone.

2. Acides amino-11 undécanoïques selon la revendication 1, caractérisés en ce que, dans la formule (I), $R^7$ représente H et E représente un radical $-\overset{R^5}{CH}-\overset{R^6}{CH}-$.

3. Esters d'acides amino-11 undécanoïques selon la revendication 1, caractérisés en ce que, dans la formule (I), $R^7$ représente un radical alkyle, linéaire ou ramifié, contenant au total de 1 à 18

atomes de carbone, de préférence de 1 à 8, et E représente un radical $-\overset{R^5}{\overset{|}{C}}H-\overset{R^6}{\overset{|}{C}}H-$.

4. Esters d'acides amino-11 undécadiène-4,8 oïques selon la revendication 1, caractérisés en ce que, dans la formule (I), $R^7$ représente un radical alkyle, linéaire ou ramifié, contenant au total de 1 à 18 atomes de carbone, de préférence de 1 à 8, et E représente un radical $-\overset{R^5}{\overset{|}{C}}=\overset{R^6}{\overset{|}{C}}-$.

5. Composées de formule (I) selon la revendication 1, dans lesquels $R^5$ et $R^6$ représentent chacun l'hydrogène, $R^1$ et $R^3$ représentent chacun, indépendamment l'un de l'autre, l'hydrogène ou un alkyle en $C_1$–$C_5$, $R^2$ représente un alkyle en $C_1$–$C_5$ et $R^4$ un alkyle en $C_1$–$C_7$, ou dans lesquels $R^3$, $R^5$ et $R^6$ représentent chacun l'hydrogène, $R^1$ et $R^2$ formant ensemble et avec l'atome de carbone qui les porte un radical cyclopentyle ou cyclohexyle, et $R^4$ représente un alkyle en $C_1$–$C_7$.

6. Composés de formule (I) selon la revendication 1, dans lesquels $R^3$, $R^5$ et $R^6$ représentent chacun l'hydrogène, $R^1$ et $R^2$ représentent chacun, indépendamment l'un de l'autre, un alkyle en $C_1$–$C_4$ ou forment ensemble et avec l'atome de carbone qui les porte un radical cyclohexyle, et $R^4$ représente un alkyle en $C_1$–$C_7$.

7. Composés de formule (I) selon la revendication 1, dans lesquels $R^3$ représente l'hydrogène et $R^4$ un radical de formule $-CH\begin{smallmatrix}R^1\\R^2\end{smallmatrix}$

8. Composé de formule (I) selon la revendication 1, dans lequel $R^1$ et $R^2$ représentent chacun $-CH_3$, $R^3$ représente $-H$, $R^4$ représente $-CH(CH_3)_2$, E représente $-CH_2CH_2-$ et $R^7$ représente un radical n-pentyle.

9. Procédé de préparation de composés de formule (I) selon la revendication (I), procédé caractérisé en ce qu'on fait réagir un aza-1 cyclododécène de formule (II)

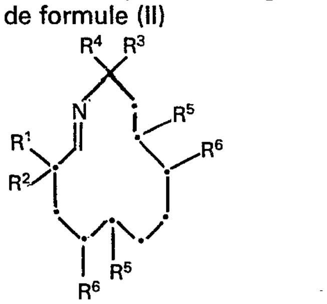

(II)

ou un aza-1 cyclododécatriène-1,5,9 de formule (III)

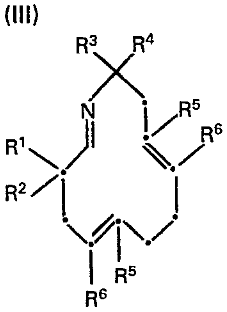

(III)

en milieu aqueux ou aqueux-organique, en présence d'un acide minéral, selon des méthodes connues, de manière à obtenir des composés de formule (IV)

$$\left[H_2N-\underset{R^4}{\overset{R^3}{C}}-CH_2-E-(CH_2)_2-E-CH_2-\underset{R^2}{\overset{R^1}{C}}-CHO\right]_n \begin{matrix}H^{\oplus}\\ X^{\ominus n}\end{matrix} \quad (IV)$$

formules dans lesquelles $R^1$ à $R^6$ et E ont les significations données à la revendication 1, X représente l'anion de l'acide minéral et n un nombre entier correspondant à la valence de X, puis on oxyde les composés de formule (IV) afin de les convertir en les acides amino-11 undécanoïques ou amino-11 undécadiène-4,8 oïques correspondants de formule (I), et éventuellement, dans un troisième étape réactionnelle, on fait réagir les acides de formule (I) ainsi obtenus, selon des méthodes connues, avec un alcool de formule $R^7$–OH dans lequel $R^7$ représente un radical alkyle, linéaire ou ramifié, contenant au total de 1 à 18 atomes de carbone, afin d'obtenir les esters de formule (I) correspondants.

10. Procédé selon la revendication 9, procédé caractérisé en ce que la réaction d'un des composés de formules (II) et (III) devant conduire aux composés de formule (IV) est effectuée en milieu aqueux, en présence d'acide sulfurique.

11. Procédé selon la revendication 9, caractérisé en ce qu'on oxyde les composés de formule (IV) en les traitant par de l'oxygène sous pression, pour les transformer en les acides amino-11 undécanoïques ou amino-11 undécadiène-4,8 oïques de formule (I) correspondants.

12. Procédé selon la revendication 11, caractérisé en ce que l'oxydation est effectuée sous une pression de 1 à 100 bars, de préférence de 3 à 20 bars, et à une température comprise entre 0 et 200 °C, de préférence entre 20 et 100 °C.

13. Procédé selon la revendication 9, pour la préparation de composés selon l'une quelconque des revendications 2 à 8, caractérisé en ce qu'on met en jeu un aza-1 cyclododécène de formule (II) ou un aza-1 cyclododécatriène-1,5,9 de formule (III) et éventuellement un alcool de formule $R^7$–OH, formules dans lesquelles $R^1$ à $R^7$ ont les significations qui leur ont été données dans les revendications 2 à 8.

**Claims:**

1. A compound of the formula I

$$H_2N-\underset{R^4}{\overset{R^3}{C}}-CH_2-E-(CH_2)_2-E-CH_2-\underset{R^2}{\overset{R^1}{C}}-CO\cdot OR^7 \quad (I)$$

wherein each of $R^1$ and $R^3$ independently is hydrogen or a straight chain or branched alkyl radical of 1 to 8 carbon atoms, and $R^3$ ist also a phenyl radical, $R^2$ is a straight chain or branched alkyl

radical of 1 to 8 carbon atoms and $R^4$ is a straight chain or branched alkyl radical of 1 to 18 carbon atoms, or $R^1$ and $R^2$ and/or $R^3$ and $R^4$, together with the carbon atom to which they are attached, form a cycloaliphatic ring containing 4 to 8 carbon atoms, and wherein each E represents one of the radicals $-\overset{R^5}{\overset{|}{C}}H-\overset{R^6}{\overset{|}{C}}H-$ and $-\overset{R^5}{\overset{|}{C}}=\overset{R^6}{\overset{|}{C}}-$ in which each of $R^5$ and $R^6$ independently is hydrogen or alkyl of 1 to 4 carbon atoms and $R^7$ is hydrogen or a straight chain or branched alkyl radical containing altogether 1 to 18 carbon atoms.

2. An 11-aminoundecanoic acid according to claim 1, wherein $R^7$ in formula I is hydrogen and E is the radical $-\overset{R^5}{\overset{|}{C}}H-\overset{R^6}{\overset{|}{C}}H-$.

3. An 11-aminoundecanoic acid ester according to claim 1, wherein $R^7$ in formula I is a straight chain or branched alkyl radical containing altogether 1 to 18, preferably 1 to 8, carbon atoms, and E is the radical $-\overset{R^5}{\overset{|}{C}}H-\overset{R^6}{\overset{|}{C}}H-$.

4. An 11-aminoundeca-4,8-dienoic acid ester according to claim 1, wherein $R^7$ in formula I is a straight chain or branched alkyl radical containing altogether 1 to 18, preferably 1 to 8, carbon atoms, and E is the radical $-\overset{R^5}{\overset{|}{C}}=\overset{R^6}{\overset{|}{C}}-$.

5. A compound of the formula I according to claim 1, wherein each of $R^5$ and $R^6$ is hydrogen, each of $R^1$ and $R^3$ independently is hydrogen or alkyl of 1 to 5 carbon atoms, $R^2$ is alkyl of 1 to 5 carbon atoms and $R^4$ is alkyl of 1 to 7 carbon atoms, or wherein each of $R^3$, $R^5$ and $R^6$ is hydrogen, $R^1$ and $R^2$ together with the carbon atom to which they are attached are cyclopentyl or cyclohexyl, and $R^4$ is alkyl of 1 to 7 carbon atoms.

6. A compound of the formula I according to claim 1, wherein each of $R^3$, $R^5$ and $R^6$ is hydrogen, each of $R^1$ and $R^2$ independently is alkyl of 1 to 4 carbon atoms or together with the carbon atom to which they are attached are cyclohexyl, and $R^4$ is alkyl of 1 to 7 carbon atoms.

7. A compound of the formula I according to claim 1, wherein $R^3$ is hydrogen and $R^4$ is the radical of the formula 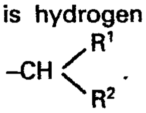 .

8. A compound of the formula I according to claim 1, wherein each of $R^1$ and $R^2$ is $-CH_3$, $R^3$ is $-H$, $R^4$ is $-CH(CH_3)_2$, E is $-CH_2CH_2$ and $R^7$ is n-pentyl.

9. A process for the manufacture of a compound of the formula I according to claim 1, which process comprises reacting a 1-azacylododecene of the formula (II)

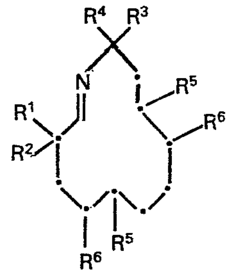

(II)

or a 1-aza-1,5,9-cyclododecatriene of the formula III

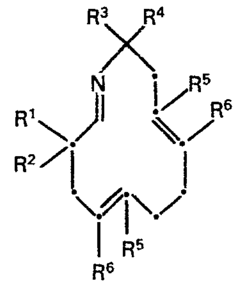

(III)

in aqueous or aqueous-organic medium, in the presence of an inorganic acid, by methods known per se, to produce compounds of the formula

$$\left[H_2N-\overset{R^3}{\underset{R^4}{C}}-CH_2-E-(CH_2)_2-E-CH_2-\overset{R^1}{\underset{R^2}{C}}-CHO\right]_n H^{\oplus} X^{\ominus n} \quad (IV)$$

in which formulae (II) to (IV) $R_1$ to $R_6$ and E are as defined in claim 1, X is the anion of the inorganic acid and n is an integer corresponding to the valency of X, and subsequently oxidising the compounds of the formula (IV) to the respective 11-amino-undecanoic acids or 11-amino-undeca-4,8-dienoic acids of the formula I, and optionally in a third reaction step, reacting these acids, by methods known per se, with an alcohol of the formula $R^7OH$, wherein $R^7$ is a straight chain or branched alkyl radical of altogether 1 to 18 carbon atoms, to produce the corresponding esters of the formula (I).

10. A process according to claim 9, wherein the reaction of one of the compounds of the formula II or III to produce compounds of the formula IV is carried out in aqueous medium in the presence of sulfuric acid.

11. A process according to claim 9, wherein the compounds of the formula IV are oxidised by treatment with oxygen under excess pressure to produce the respective 11-aminoundecanoic acids or 11-aminoundeca-4,8-dienoic acids of the formula I.

12. A process according to claim 11, wherein the oxidation is carried out at a pressure of 1 to 100 bar, preferably 3 to 20 bar, and at a temperature in the range from 0° to 200 °C, preferably form 20° to 100 °C.

13. A process according to claim 9 for the manufacture of compounds according to claims 2 to 8,

which process comprises the use of a 1-aza-cyclododecene of the formula II or a 1-aza-1,5,9-cyclododecatriene of the formula III and optionally an alcohol of the formula $R^7$–OH, in which formulae II, III and $R^7$–OH $R^1$ to $R^7$ are as defined in claims 2 to 8.